# EUROPEAN PATENT SPECIFICATION

(11) **EP 1 594 553 B1**
(45) Date of publication and mention of the grant of the patent: **08.08.2007**
(21) Application number: 04709678.9
(22) Date of filing: 10.02.2004
(51) Int. Cl.: A61L 15/28, A61L 15/46

(54) **DISPOSABLE DIAPER FOR COMBATING DIAPER RASH**
WEGWERFWINDEL ZUR BEKÄMPFUNG VON WINDELAUSSCHLAG
COUCHE JETABLE PERMETTANT DE PREVENIR L'ERYTHEME FESSIER

(30) Priority: 21.02.2003 US 371491; 13.01.2004 US 757786
(43) Date of publication of application: 16.11.2005
(73) Proprietor: The Cupron Corporation, New York, NY 10017 (US)
(72) Inventor: GABBAY, Jeffrey, 92142 Jerusalem (IL)
(74) Representative: Fairbairn, Angus Chisholm
(86) International application number: PCT/IL2004/000127
(87) International publication number: WO 2004/073756

(56) References cited:
- WO-A-01/81671
- WO-A-98/06508
- WO-A-98/06509
- GB-A- 415 213
- US-A- 4 317 856
- US-A- 4 675 014
- US-A- 5 856 248

## Description

The present invention relates to a disposable diaper for combating diaper rash. More particularly, the present invention relates to a disposable diaper for combating diaper rash and a method for the manufacture thereof wherein said diaper comprises a plurality of fibers coated with an anti-bacterial and anti-fungal Cu⁺⁺ cationic, water insoluble form of copper.

Paper products having various forms of copper incorporated therein have been described in the patent literature and thus e.g. in US Patent 1,947,451 published in February 1934 there is described a paper sheet having fungicidal and bacteriacidal properties carrying a predetermined amount of copper orthophenyl phenate precipitate.

Similarly many patents from decades ago such as US Patents 1,747,232; 1,846,185; 1,946,952; 1,988,231; 2,749,256; 3,492,464 and 3,713,963 disclose the incorporation of different forms of copper into paper however all of said patents as well as US Patent 1,947,451 teach the incorporation of soluble forms of copper for other purposes and do not teach or suggest the use of an anti-bacterial and anti-fungal Cu⁺⁺ cationic, water insoluble form of copper or the use thereof for preparing disposable paper diapers.

It is to be noted that the water soluble forms of copper taught by said patents would be totally inappropriate for use in the present invention since such copper would be washed away with urination and would not be available to achieve the desired effect of combating diaper rash.

Thus while the use of copper as a biocide in paper is not entirely new, the addition of a water insoluble cationic species in the form described in the present application is new.

Thus according to the present invention there is now provided a disposable paper diaper for combating diaper rash, said diaper comprising a plurality of fibers coated with an anti-bacterial and anti-fungal Cu⁺⁺ cationic, water-insoluble form of copper.

In preferred embodiments of the present invention said fibers are cellulosic fibers.

In especially preferred embodiments of the present invention said coated fibers are disposed in said diaper as randomly scattered fibers in a paper layer.

Preferably said coated fibers are dispersed in said diaper in a layer positioned in said diaper to rest against the skin of the user.

In another aspect of the present invention there is provided a method for the manufacture of a disposable paper diaper for combating diaper rash comprising incorporating a plurality of fibers coated with an anti-bacterial and anti-fungal, Cu⁺⁺ cationic, water-insoluble form of copper in an inner layer of said disposable diaper.

In a further aspect of the present invention there is provided a disposable diaper liner for combating diaper rash, said diaper liner comprising a plurality of fibers coated with an anti-bacterial and anti-fungal Cu⁺⁺ cationic, water-insoluble form of copper.

In both WO 98/06508 and WO 98/06509 there are taught various aspects of a textile with a full or partial metal or metal oxide plating directly and securely bonded to the fibers thereof, wherein metal and metal oxides, including copper, are bonded to said fibers.

More specifically, in WO 98/06509 there is provided a process comprising the steps of: (a) providing a metallized textile, the metallized textile comprising: (i) a textile including fibers selected from the group consisting of natural fibers, synthetic cellulosic fibers, regenerated fibers, acrylic fibers, polyolefin fibers, polyurethane fibers, vinyl fibers, and blends thereof, and (ii) a plating including materials selected from the group consisting of metals and metal oxides, the metallized textile characterized in that the plating is bonded directly to the fibers; and (b) incorporating the metallized textile in an article of manufacture.

In the context of said invention the term "textile" includes fibers, whether natural (for example, cotton, silk, wool, and linen) or synthetic yarns spun from those fibers, and woven, knit, and non-woven fabrics made of those yarns. The scope of said invention includes all natural fibers; and all synthetic fibers used in textile applications, including but not limited to synthetic cellulosic fibers (i.e., regenerated cellulose fibers such as rayon, and cellulose derivative fibers such as acetate fibers), regenerated protein fibers, acrylic fibers, polyolefin fibers, polyurethane fibers, and vinyl fibers, but excluding nylon and polyester fibers, and blends thereof.

Said invention comprised application to the products of an adaptation of technology used in the electrolyses plating of plastics, particularly printed circuit boards made of plastic, with metals. See, for example, Encyclopedia of Polymer Science and Engineering (Jacqueline I. Kroschwitz, editor), Wiley and Sons, 1987, vol. IX, pp 580-598. As applied to textiles, this process included two steps. The first step was the activation of the textile by precipitating catalytic noble metal nucleation sites on the textile. This was done by first soaking the textile in a solution of a low-oxidation-state reductant cation, and then soaking the textile in a solution of noble metal cations, preferably a solution of Pd⁺⁺ cations, most preferably an acidic PdCl₂ solution. The low-oxidation-state cation reduces the noble metal cations to the noble metals themselves, while being oxidized to a higher oxidation state. Preferably, the reductant cation is one that is soluble in both the initial low oxidation state and the final high oxidation state, for example Sn⁺⁺, which is oxidized to Sn⁺⁺⁺⁺, or Ti⁺⁺⁺, which is oxidized to Ti⁺⁺⁺⁺.

The second step was the reduction, in close proximity to the activated textile, of a metal cation whose reduction was catalyzed by a noble metal. The reducing agents used to reduce the cations typically were molecular species, for example, formaldehyde in the case of Cu⁺⁺. Because the reducing agents were oxidized, the metal cations are termed "oxidant cations" herein. The metallized textiles thus produced were characterized in that their metal plating was bonded directly to the textile fibers.

In WO 98/06508 there is described and claimed a composition of matter comprising:
(a) a textile including fibers selected from the group consisting of natural fibers, synthetic cellulosic fibers, regenerated protein fibers, acrylic fibers, polyolefin fibers, polyurethane fibers, vinyl fibers, and blends thereof; and
(b) a plating including materials selected from the group consisting of metals and metal oxides;
   the composition of matter characterized in that said plating is bonded directly to said fibers.

Said publication also claims a composition of matter comprising:
(a) a textile including fibers selected from the group consisting of natural fibers, synthetic cellulosic fibers, regenerated protein fibers, acrylic fibers, polyolefin fibers, polyurethane fibers, vinyl fibers, and blends thereof; and
(b) a plurality of nucleation sites, each of said nucleation sites including at least one noble metal;
the composition of matter characterized by catalyzing the reduction of at least one metallic cationic species to a reduced metal, thereby plating said fibers with said reduced metal.

In addition, said publication teaches and claims processes for producing said products.

A preferred process for preparing a metallized textile according to said publication comprises the steps of:
a) selecting a textile, in a form selected from the group consisting of yarn and fabric, said textile including fibers selected from the group consisting of natural fibers, synthetic cellulosic fibers, regenerated protein fibers, acrylic fibers, polyolefin fibers, polyurethane fibers, vinyl fibers, and blends thereof;
b) soaking said textile in a solution containing at least one reductant cationic species having at least two positive oxidation states, said at least one cationic species being in a lower of said at least two positive oxidation states;
c) soaking said textile in a solution containing at least one noble metal cationic species, thereby producing an activated textile; and
d) reducing at least one oxidant cationic species in a medium in contact with said activated textile, thereby producing a metallized textile.

While the metallized fabrics produced according to said publications are effective acaricides, it was found that they are also effective in preventing and/or treating bacterial, fungal and yeast infections which afflict various parts of the human body and that therefore the incorporation of at least a panel of a metallized textile material in an article of clothing can have extremely beneficial effect.

Thus, in US Patent 6,124,221 there is described and claimed an article of clothing having anti-bacterial, anti-fungal, and anti-yeast properties, comprising at least a panel of a metallized textile, the textile including fibers selected from the group consisting of natural fibers, synthetic cellulosic fibers, regenerated protein fibers, acrylic fibers, polyolefin fibers, polyurethane fibers, vinyl fibers, and blends thereof, and having a plating including an anti-bacterial, anti-fungal and anti-yeast effective amount of at least one oxidant cationic species of copper.

In said specification there was described that said article of clothing was effective against *Tinea Pedis,* against *Candida Albicans,* against *Thrush* and against bacteria causing foot odor, selected from the group consisting of *brevubacterium, acinetobacter, micrococcus* and combinations thereof.

Thus, said invention was especially designed for preparation of articles such as underwear and articles of hosiery.

In WO 01/81671 there is described that textile fabrics incorporating fibers coated with a cationic form of copper are also effective for the inactivation of antibiotic resistant strains of bacteria and said cationic species of copper preferably comprises Cu⁺⁺ ions.

It is to be noted however that textile chemistry is different than paper chemistry and it was not obvious to apply the teachings of said applications and patents which were directed to textile fabrics to paper chemistry to produce the diapers of the present invention.

More specifically it is to be noted that normal paper mulch is usually in an alkaline state with a pH which can vary from 8 to 11. While this atmosphere allows a reduction of copper to a cationic state to occur in an oxidation reduction process, the elements and the pH of the mulch will inhibit a full chemical reaction. The reduction process will upset the malleability of the mulch and the inhibition of the full reaction will in turn cause a limit to the biocidal and fungicidal quality of the mulch.

In order to have an effective level of biocidal and fungicidal activity and in order not to upset the proper production of paper, it was found according to the present invention that a fiber prepared with a plating of a cationic species of copper on it could be added to the mulch in the final stages of production without disturbing the production of biocidal and fungicidal qualities of the mulch. Since the copper on the fiber does not react to an alkaline solution or atmosphere, it was found that the full biocidal and fungicidal qualities were retained.

Thus, none of the above publications teach or suggest the subject matter of the present invention.

Furthermore as will be noted hereinafter in the production of paper acrylic glue is added to act as a binder for the paper and there was the possibility that the acrylic binder would encapsulate the copper compound and prevent the copper ions from being effective as an anti-bacterial and anti-fungal agent. Surprisingly this was found not to be the case and for this reason also the diapers of the present invention and their ability to combat diaper rash is unexpected and neither taught or suggested by the prior art.

While the invention will now be described in connection with certain preferred embodiments in the following examples and with reference to the attached figures, so that aspects thereof may be more fully understood and appreciated, it is not intended to limit the invention to these particular embodiments. On the contrary, it is intended to cover all alternatives, modifications and equivalents as may be included within the scope of the invention as defined by the appended claims. Thus, the following examples which include preferred embodiments will serve to illustrate the practice of this invention, it being understood that the particulars shown are by way of example and for purposes of illustrative discussion of preferred embodiments of the present invention only and are presented in the cause of providing what is believed to be the most useful and readily understood description of formulation procedures as well as of the principles and conceptual aspects of the invention.
In the drawings:

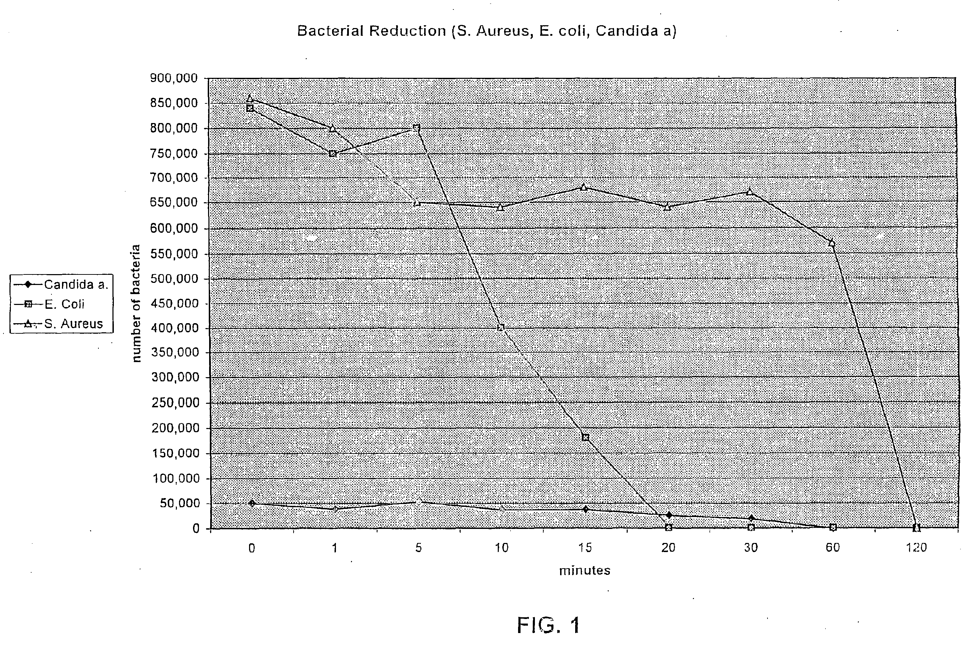

FIG. 1 is a graphical representation of bacterial and fungal reduction with time using a sheet of paper comprising a plurality of fibers according to the present invention.

### Example 1

### a) Preparation of the fibers

i) Fibers were exposed to a tin dichloride solution and then rinsed in plain water;
ii) Fibers were exposed to a palladium solution and then rinsed in plain water;
iii) Fibers were exposed to a copper sulfate chelating solution;
iv) Fibers were exposed to a reducing agent; and
v) Fibers were allowed to dwell for no less than 2 minutes or until all fibers were plated by a dark brown form of copper.

### b) Preparation of a paper incorporating said fibers

50 grams (dry weight) of a soft fibrous carton was prepared by chopping it into small pieces. The cut carton was placed in a soapy solution and heated to about 80°C and allowed to remain at that state for about 15 minutes to facilitate removal of any binders in the slurry.

The slurry was then rinsed with cold water and strained to remove excess liquid. The slurry was then placed in blender with a small amount of water and allowed to mix until a very fine slurry was obtained. The moist fine slurry was then divided into three small batches by weight each weighing about 90 grams after removal of excess liquid. The three batches were marked "A", "B", and "C". To sample A, 5 grams of finely chopped Cu coated cellulose fibers was added and allowed to mix in a blender. To sample B, 5 grams of Cuprous Oxide power was added and also allowed to mix in a blender. The Cuprous Oxide power particle size was 4 to 5 microns. Sample C was designated as a control to which no copper product was added. When a fine slurry of each was obtained in its own blender, 5 grams of an acrylic glue was added to each to act as a binder for the paper.

A small amount of each slurry was placed between layers of absorbent paper and run through a squeeze roll at about 8 bars of pressure. This proved to be enough pressure to remove almost all the liquid in the slurry and still leave a flat paper. The paper was then dried using a hot air dryer.

### Example 2

The produced paper samples were tested on Gram+ and Gram- bacteria, as well as a common fungus, Candida Albicans (which is known to have resistance to copper). The test methods used to measure efficacy were a west agar system and a diffusion system.

1 gram of each sample was placed in a sterile tube, containing 20 ml of peptone water. Each tube was inoculated with a suspension of the test microorganism. The control sample was prepared with 20 ml peptone water. The inoculated tubes were incubated at 30°C (Candida Albicans) or 35°C (Gram+, Gram-) for 2, 4, and 24 hours. The number of surviving bacteria was determined using the pour plate method.

### Experimental Conditions:

| Test Temperature | Room |
|---|---|
| Test Microorganisms | E. coli ATCC 8739. Staphylococcus aureus ATCC 6538, Candida Albicant ATCC 10231 |
| Contact Time | 0, 2, 4 and 24 hours |
| Counting Procedure | Pour Plate Count |
| Test Media | Tryptic Soy Agar, Difco |
| Temperature of Incubation | 30°C, 35°C |
| Incubation Period | 24-48 hours |

### Evaluation of Bactericidal Activity of Cu⁺⁺ Treated Matrix

### Test Results:

| **Test Microorganism** | **CFU/ml** | | | |
|---|---|---|---|---|
| | **Escherichia coli ATCC 8739** | | | |
| Exposure | 0 hours | 2 hours | 4 hours | 24 hours |
| Sample ID | | | | |
| A (treated) | 1.4x10⁴ | 3 | 0 | N/A |
| B (treated) | 1.3x10⁴ | 580 | 3 | |
| C (untreated) | 1.4x10⁴ | 6.6x10³ | 7.0x10³ | |
| Peptone Water | 1.5x10⁴ | 5.7x10³ | 790 | |

| **Test Microorganism** | **CFU/ml** | | | |
|---|---|---|---|---|
| | **Staphylococcus aureus ATCC 6538** | | | |
| Exposure | 0 hours | 2 hours | 4 hours | 24 hours |
| Sample ID | | | | |
| A (treated) | 5.6x10³ | 5.8x10³ | 5.4x10³ | 0 |
| B (treated) | 5.2x10³ | 4.6x10³ | 4.7x10³ | 690 |
| C (untreated) | 5.3x10³ | 5.2x10³ | 8.0x10³ | 9.5x10³ |
| Peptone Water | 5.2x10³ | 7.2x10³ | 7.5x10³ | 8.8x10³ |

### Evaluation of Fungicidal Activity of Cu⁺⁺ Treated Matrix

| **Test Microorganism** | **CFU/ml** | | | |
|---|---|---|---|---|
| | **Candida albicans ATCC 10231** | | | |
| Exposure | 0 hours | 2 hours | 4 hours | 24 hours |
| Sample ID | | | | |
| A (treated) | 20x10³ | 14x10³ | 1.1x10³ | 150 |
| B (treated) | 2.1x10³ | 1.7x10³ | 1.5x10³ | 850 |
| C (untreated) | 2.9x10³ | 1.5x10³ | 1.5x10³ | 1.1x10⁴ |
| Peptone Water | 2.8x10³ | 1.8x10³ | 1.7x10³ | 9.8x10⁴ |

As is known, diaper rash is caused initially by sensitivity to ammonia from urine however is subsequently aggravated by fungicidal infection and/or by both gram negative and gram positive bacteria and the above tests show the effectiveness of the Cu⁺⁺ treated paper to significantly reduce the concentration of E. coli which is a gram negative bacteria as well as to reduce concentration of staphylococcus aureus which is a gram positive bacteria and to reduce the concentration of the fungus, Candida Albicans.

Thus, both soiled diapers and clean diapers according to the present invention will be effective to release anti-bacterial and anti-fungal Cu⁺⁺ ions since urine will act as a medium for the transmittal of the ions to the skin of the wearer and even with fresh diapers, the moisture layer of the skin against the diaper will act as said medium.

It is to be noted that the test method used above followed AATCC Test Method 100 which is a quantitative test that measures the level of product efficacy and tells you how much bacteria was killed. Since Test Method 100 calls for a two hour incubation the test results show efficacy levels only after two hours.

In order to further determine bacterial reduction over time several sheets of paper prepared according to Example 1 were tested at 0 minutes, 1, minute, 5 minutes, 10 minutes, 15 minutes, 20 minutes, 25 minutes, 30 minutes, 1 hour and 2 hours and the results are set forth in the graph presented as Figure 1. As will be noted, in these tests Staph Aureus showed total reduction after 2 hours while Candida showed reduction after 1 hour with E. coli showing full reduction after only 20 minutes.

As will be remembered, in the days of cloth diapers, there were marketed diaper liners and it will be understood that the technology of the present invention can be applied to the preparation of diaper liners to be used in conjunction with disposable diapers which have not been produced according to the present invention.

It will be evident to those skilled in the art that the invention is not limited to the details of the foregoing illustrative examples and that the present invention may be embodied in other specific forms without departing from the essential attributes thereof, and it is therefore desired that the present embodiments and examples be considered in all respects as illustrative and not restrictive, reference being made to the appended claims, rather than to the foregoing description, and all changes which come within the meaning and range of equivalency of the claims are therefore intended to be embraced therein.

## Claims

1. A disposable paper diaper formed from paper mulch for combating diaper rash, said diaper comprising a plurality of finely chopped fibers coated with an anti-bacterial and anti-fungal cationic, water-insoluble form of copper comprising Cu⁺⁺ directly bound to the fibers, which fibers have been added to said paper mulch and which fibers release Cu⁺⁺ ions when in contact with a fluid.

2. A disposable paper diaper for combating diaper rash according to claim 1 wherein said fibers are cellulosic fibers.

3. A disposable paper diaper for combating diaper rash according to claim 1 wherein said coated fibers are disposed in said diaper as randomly scattered fibers in a paper layer.

4. A disposable paper diaper for combating diaper rash according to claim 1 wherein said coated fibers are dispersed in a paper layer positioned in said diaper to rest against the skin of the user.

5. A method for the manufacture of a disposable paper diaper formed from paper mulch for combating diaper rash comprising incorporating into said paper mulch a plurality of finely chopped fibers coated with an anti-bacterial and anti-fungal, cationic, water-insoluble form of copper comprising Cu⁺⁺ directly bound to the fibers, which fibers release Cu⁺⁺ ions when in contact with a fluid, said fibers being provided in an inner layer of said disposable diaper.

6. A method according to claim 5 wherein said fibers are cellulosic fibers.

7. A disposable diaper liner formed from paper mulch for combating diaper rash, said diaper liner comprising a plurality of finely chopped fibers coated with an anti-bacterial and anti-fungal cationic, water-insoluble form of copper comprising Cu⁺⁺ directly bound to the fibers, which fibers have been added to said paper mulch and which fibers release Cu⁺⁺ ions when in contact with a fluid.

## Patentansprüche

1. Wegwerfpapierwindel, erzeugt aus Papiermulch, zur Bekämpfung von Windelausschlag, wobei die Windel eine Mehrzahl von fein zerhackten Fasern, beschichtet mit einer antibakteriellen und antimykotischen kationischen, wasserunlöslichen Form von Kupfer, umfassend Cu⁺⁺, direkt gebunden an die Fasern, umfaßt, welche Fasern zu dem Papiermulch hinzugegeben worden sind und welche Fasern Cu⁺⁺-Ionen freisetzen, wenn sie in Kontakt mit einer Flüssigkeit sind.

2. Wegwerfpapierwindel zur Bekämpfung von Windelausschlag nach Anspruch 1, wobei die Fasern Cellulosefasern sind.

3. Wegwerfpapierwindel zur Bekämpfung von Windelausschlag nach Anspruch 1, wobei die beschichteten Fasern als statistisch verstreute Fasern in einer Papierschicht in der Windel angeordnet sind.

4. Wegwerfpapierwindel zur Bekämpfung von Windelausschlag nach Anspruch 1, wobei die beschichteten Fasern in einer Papierschicht dispergiert sind, die in der Windel positioniert ist, um an der Haut des Benutzers zu liegen.

5. Verfahren für die Herstellung einer Wegwerfpapierwindel, erzeugt aus Papiermulch, zur Bekämpfung von Windelausschlag, umfassend Einbringen einer Mehrzahl von fein zerhackten Fasern, beschichtet mit einer antibakteriellen und antimykotischen, kationischen, wasserunlöslichen Form von Kupfer, umfassend Cu⁺⁺, direkt gebunden an die Fasern, in den Papiermulch, welche Fasern Cu⁺⁺-Ionen freisetzen, wenn sie in Kontakt mit einer Flüssigkeit sind, wobei die Fasern in einer Innenschicht der Wegwerfwindel bereitgestellt werden.

6. Verfahren nach Anspruch 5, wobei die Fasern Cellulosefasern sind.

7. Wegwerfwindeleinlage, erzeugt aus Papiermulch, zur Bekämpfung von Windelausschlag, wobei die Windeleinlage eine Mehrzahl von fein zerhackten Fasern, beschichtet mit einer antibakteriellen und antimykotischen kationischen, wasserunlöslichen Form von Kupfer, umfassend Cu⁺⁺, direkt gebunden an die Fasern, umfaßt, welche Fasern zu dem Papiermulch hinzugegeben worden sind und welche Fasern Cu⁺⁺-Ionen freisetzen, wenn sie in Kontakt mit einer Flüssigkeit sind.

## Revendications

1. Couche en papier jetable formée à partir d'un paillage papier pour combattre l'érythème fessier, ladite couche comprenant plusieurs fibres finement coupées recouvertes avec une forme de cuivre antibactérien et antifongique, cationique, non soluble dans l'eau comprenant Cu⁺⁺ directement lié aux fibres, lesquelles fibres ont été ajoutées audit paillage papier et lesquelles fibres libèrent des ions Cu⁺⁺ quand elles sont en contact avec un fluide.

2. Couche en papier jetable pour combattre l'érythème fessier selon la revendication 1, dans laquelle lesdites fibres sont des fibres cellulosiques.

3. Couche en papier jetable pour combattre l'érythème fessier selon la revendication 1, dans laquelle lesdites fibres recouvertes sont disposées dans ladite couche comme des fibres éparpillées au hasard dans une nappe de papier.

4. Couche en papier jetable pour combattre l'érythème fessier selon la revendication 1, dans laquelle lesdites fibres recouvertes sont dispersées dans une nappe de papier positionnée dans ladite couche pour être en contact avec la peau de l'utilisateur.

5. Procédé pour la fabrication d'une couche en papier jetable formée à partir d'un paillage papier pour combattre l'érythème fessier comprenant le fait d'incorporer dans ledit paillage papier plusieurs fibres finement coupées recouvertes avec une forme de cuivre antibactérien et antifongique, cationique, non soluble dans l'eau comprenant Cu⁺⁺ directement lié aux fibres, lesquelles fibres libèrent des ions Cu⁺⁺ quand elles sont en contact avec un fluide, lesdites fibres étant disposées dans une nappe intérieure de ladite couche jetable.

6. Procédé selon la revendication 5, dans lequel lesdites fibres sont des fibres cellulosiques.

7. Garniture de couche jetable formée à partir d'un paillage papier pour combattre l'érythème fessier, ladite garniture de couche comprenant plusieurs fibres finement coupées recouvertes avec une forme de cuivre antibactérien et antifongique, cationique, non soluble dans l'eau comprenant Cu⁺⁺ directement lié aux fibres, lesquelles fibres ont été ajoutées audit paillage papier et lesquelles fibres libèrent des ions Cu⁺⁺ quand elles sont en contact avec un fluide.
